# EUROPEAN PATENT APPLICATION

(11) **EP 4 501 394 A1**
(43) Date of publication of application: **05.02.2025**
(21) Application number: 23781007.2
(22) Date of filing: 30.03.2023
(51) Int. Cl.: A61N 1/05, A61N 1/08, A61N 1/36

(54) **STIMULUS APPLICATION SYSTEM, IMPLANT DEVICE, CONTROL DEVICE, METHOD FOR CONTROLLING CONTROL DEVICE, AND PROGRAM**

(30) Priority: 31.03.2022 WO PCT/JP2022/016747
(71) Applicant: Inopase Inc., Tokyo 103-0023 (JP)
(72) Inventor: WANG, Yen Po, Tokyo 103-0023 (JP); SUGIMOTO, Munemasa, Tokyo 103-0023 (JP)
(74) Representative: Huebner, Stefan Rolf
(86) International application number: PCT/JP2023/013414
(87) International publication number: WO 2023/190999

(57) **Abstract**

Provided is an implant device 10 implanted in an object body which is an animal including a human being, and being communicably connected to a controller device 20 arranged outside of the object body, wherein the implant device 10 detects an electrical signal as a physiological signal at a predetermined part in the object body, transmits detection information representing time variation of the electrical signal, receives, from the controller device 20, a stimulation instruction representing a stimulus to be applied to the object body, and applies an electrical stimulus to a predetermined part in the object body, on the basis of the stimulation instruction.

## Description

### Technical Field

The present disclosure relates to a stimulus application system, an implant device, a controller device, a method for controlling a controller device, and a program.

### Background Art

Patent Document 1 discloses a neural implant device which comprises a circuit configured to receive an input signal and to generate an electrical signal based on the received input signal.

### Prior Arts

### Patent Document

Patent Document 1: Japanese Unexamined Patent Publication (Kokai) No. 2019-503809

### Summary

However, the above-mentioned conventional neural implant device has drawbacks that control based on the internal physiological signals is not performed, and thus, stimulus corresponding to the status of the object person may not be applied.

The present disclosure has been created in view of the above, and one of the objectives of the present disclosure is to provide a stimulus application system, an implant device, a controller device, a method for controlling a controller device, and a program, capable of applying stimulus corresponding to the status of the object.

In order to solve the above drawbacks of the prior arts, an aspect of the present disclosure is an implant device implanted in an object body which is an animal including a human being, and being wirelessly communicably connected to a controller device arranged outside of the object body, wherein the implant device comprises: a circuitry for detection which detects an electrical signal representing a physiological signal at a predetermined part in the object body, a circuitry for transmission/reception which transmits detection information representing time variation of the detected electrical signal, and receives, from the controller device, a stimulation instruction representing a stimulus to be applied to the object body, and a circuitry for application which applies an electrical stimulus to a predetermined part in the object body, on the basis of the stimulation instruction received by the circuitry for transmission/reception.

Further, in order to solve the above drawbacks of the prior arts, another aspect of the present disclosure is a controller device arranged outside of an object body which is an animal including a human being, and wirelessly communicably connected to an implant device implanted in the object body, wherein the implant device detects an electrical signal as a physiological signal at a predetermined part in the object body, and the controller device comprises: a circuitry for reception which receives the detection information transmitted by the implant device and representing a detection result of the electrical signal, a circuitry for determination which determines type of stimulus to be applied to the object body by the implant device on the basis of the received detection information, and a circuitry for transmission which transmits a stimulation instruction representing the determined stimulus to the implant device.

According to the present disclosure, stimulus corresponding to the status of an object can be applied.

### Brief Description of Drawings

FIG. 1 is a structural block diagram showing an example of a stimulus application system according to an aspect of the present disclosure.
FIG. 2 is a functional block diagram showing an example of a controller device according to an aspect of the present disclosure.
FIG. 3 is a flowchart showing an operation example of a stimulus application system according to an aspect of the present disclosure.
FIG. 4 is a functional block diagram showing another example of a controller device according to an aspect of the present disclosure.
FIG. 5 is an explanatory view showing an example of frequency domain information of detection information detected by a stimulus application system according to an aspect of the present disclosure.
FIG. 6 is an explanatory view showing an example of stimulus setting information to be used by a stimulus application system according to an aspect of the present disclosure.
FIG. 7 is an explanatory view showing an example of a signal to be processed by a stimulus application system according to an aspect of the present disclosure.
FIG. 8 is another explanatory view showing an example of a signal to be processed by a stimulus application system according to an aspect of the present disclosure.

### Aspects of Disclosure

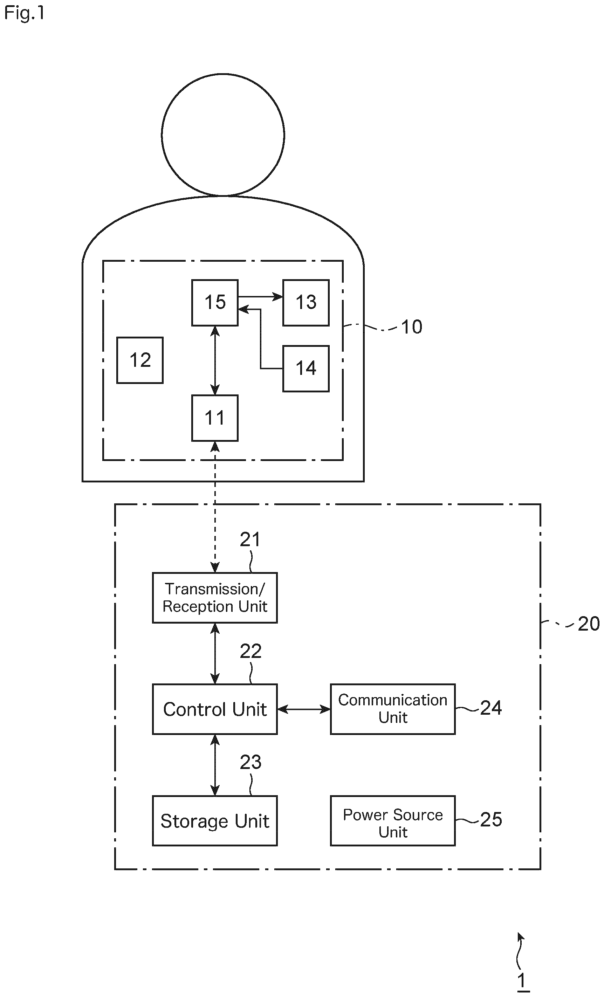

Aspects of the present disclosure will be explained with reference to the drawings. As exemplified in FIG. 1, a stimulus application system 1 according to an aspect of the present disclosure includes an implant device 10 implanted in an object body which is an animal including a human being, and a controller device 20 which arranged outside of the object body.

Here, the implant device 10 includes a transmission/reception unit 11, a power supply unit 12, a stimulus circuit unit 13, a sensor unit 14, and a processor unit 15. The controller device 20 includes a transmission/reception unit 21, a control unit 22, a storage unit 23, a communication unit 24, and a power source unit 25.

The transmission/reception unit 11 of the implant device 10 transmits data to the controller device 20 arranged outside of the object body, in accordance with instructions input from the processor unit 15. Further, the transmission/reception unit 11 receives data from the controller device 20, and outputs the data to the processor unit 15. Here, a widely known data transmission/reception method can be adopted, such as NFC, Wi-Fi, Bluetooth (registered trademark), RFID wireless communication standard, and the like. According to an example of the present aspect, the transmission/reception unit 11 can receive wireless power transfer from the controller device 20, and output the received power to the power supply unit 12.

The power supply unit 12 is provided with a battery B, and supplies power to each unit of the implant device 10. According to an example of the present aspect, the battery B provided in the power supply unit 12 can be a secondary battery, so that the power supply unit 12 receives power input from the transmission/reception unit 11 and charges the battery B.

The stimulus circuit unit 13 is under control of the processor unit 15, and applies a stimulus to the object body through electrodes arranged at predetermined parts (hereinbelow, referred to as stimulation parts) in the object body. Herein, the stimulation part where the electrode is arranged is a part to which stimulus to nerve etc., can be applied, as used in Spinal Cord Stimulation, Sacral Neuro Modulation, Vagus Nerve Stimulation, Deep Brain Stimulation, and the like. The stimulation parts can be selected in accordance with the type of stimulus to be applied to the object body. For the arrangement of the electrodes of the stimulus circuit unit 13, arrangements widely known as the arrangements used for the above-mentioned various stimulation methods can be adopted. Therefore, detailed explanation therefor is omitted here. Further, the stimulus may be a periodical electric signal, a single pulse signal, etc., and an amplitude, a frequency, a duration, a pulse width, and the like of the signal can be controlled by the processor unit 15 which is explained below.

The sensor unit 14 detects electric signals representing physiological signals of the object body (for example, signals representing the physiological signals at the detection parts of the object body, by the magnitude of potential thereof), through the electrodes arranged at predetermined parts (hereinbelow, referred to as detection parts) in the object body. Here, the physiological signal includes membrane potential, Nerve action potential, Organ pressure, Tissue impedance, temperature, and other signals acting as biomarkers, in the object body, and the physiological signal can be selected in accordance with a rule predetermined depending on the type of stimulus to be applied. Further, the stimulation part and the detection part may be different parts, adjacent (comparatively close) parts, or the same part.

The processor unit 15 includes a program controller device such as a CPU, and a storage device such as a memory. The processor unit 15 transmits detection information representing the electrical signal detected by the sensor unit 14 (for example, in case that the electrical signal represents the physiological signal at the detection part in the object body by the magnitude of its potential, the detection information is information representing the magnitude of the potential), through the transmission/reception unit 11, to the controller device. The processor unit 15 can transmit the detection information representing the detected electrical signal every time that the electrical signal detected, or can store the information representing the electrical signals detected for a plurality of times in a memory, etc., and then, transmit the information representing the detection information and stored in the memory, at a predetermined time, through the transmission/reception unit 11, to the controller device 20.

Namely, for example, the detection information may include information representing an electrical signal as a result of one-time detection, or may include information representing a plurality of electrical signals as a result of a plurality of times of detection (representing detection information representing the time variation of the detected electrical signal). In case that the information representing the electrical signals as a result of a plurality of times of the detection is included, in view of the transformation to frequency-domain information which is to be performed later by the controller device 20, information representing electrical signals as a result of 2ⁿ times of detection (n being a natural number of 1 or more) may be included in the detection information.

In this case, the processor unit 15 repeatedly obtains electrical signals representing physiological signals at a predetermined part in the object body, through the detection by the sensor unit 14 at predetermined time interval for 2ⁿ times (n being a natural number of 1 or more), and stores information representing the electrical signals as results of the detection. Then, the processor unit 15 transmits the stored detection results of the electrical signals by the 2ⁿ times of detection to the controller device 20 as detection information representing the time variation of the electrical signal.

Further, the processor unit 15 accepts an instruction (stimulation instruction) received through the transmission/reception unit 11 from the controller device 20. In accordance with the stimulation instruction, the processor unit 15 determines the parameter such as the frequency and the intensity (amplitude) of the electrical signal to be applied to the object body as a stimulus, the pulse width (in case that the electrical signal is a pulse signal), and in addition, the time when the stimulus is to be applied, the duration of the stimulus, and the like. Then, the processor unit 15 controls the stimulus circuit unit 13 so that the stimulus of the electrical signal defined by the determined parameters is applied. Operations of the processor unit 15 are to be described later.

The controller device 20 is arranged at a position outside of the object body which is an animal including a human being, and wirelessly communicable with the implant device 10. For example, the controller device 20 is wearable (usually referred to as a wearable device), and is attached on the body surface of the object body.

The transmission/reception unit 21 receives the detection information transmitted by the implant device 10, and outputs the received detection information to the control unit 22. In accordance with the instruction input from the control unit 22, the transmission/reception unit 21 transmits the instructed information to the implant device 10. Further, according to an example of the present aspect, the transmission/reception unit 21 can wirelessly supply power to the implant device 10.

The control unit 22 is a program-controlled device such as a CPU, etc., and operates in accordance with a program stored in the storage unit 23. According to an example of the present aspect, the control unit 22 receives the detection information transmitted by the implant device 10, from the transmission/reception unit 21. Then, on the basis of the received detection information, the control unit 22 determines the details of the stimulus to be applied from the implant device 10 to the object body, and instructs the transmission/reception unit 21 to transmit stimulation instruction representing the determined stimulus to the implant device 10. Operations of the control unit 22 will be described in detail below.

The storage unit 23 is a memory device, etc., which stores a program to be executed by the control unit 22. The program may be provided by being stored in a computer readable and non-transitory storage medium and copied to and stored in the storage unit 23. Further, the storage unit 23 can operate as a work memory of the control unit 22.

The communication unit 24 is a network interface which performs data communication through, for example, a wireless LAN, a cellular phone network. In accordance with the instruction input from the control unit 22, the communication unit 24 transmits data, through a communication means such a network, etc., to an instructed destination. Further, the communication unit 24 outputs the data received through the communication means such as a network, etc., to the control unit 22.

The power source unit 25 supplied power to each part of the controller device 20. Also, when the transmission/reception unit 21 wirelessly supplies power to the implant device 10, the power source unit 25 supplies power for the power supply.

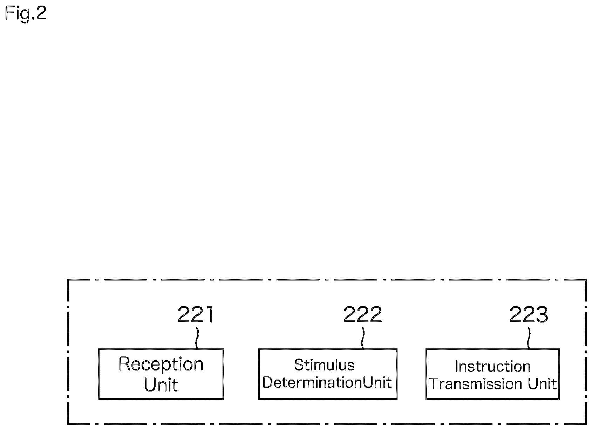

Next, an operation example of the control unit 22 of the controller device 20 will be explained. According to an example of the present aspect, the control unit 22 executes the program stored in the storage unit 23, and thereby, as exemplified in FIG. 2, the control unit 22 functionally comprises a reception unit 221, a stimulus determination unit 222, and an instruction transmission unit 223.

The reception unit 221 receives detection information transmitted by the implant device 10, the detection information being a detection result of the electrical signal detected by the implant device 10 as a physiological signal of the object body.

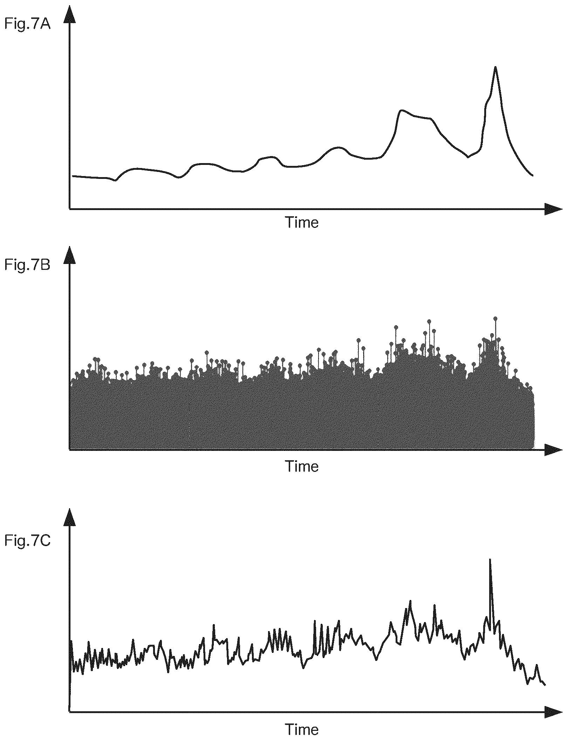

On the basis of the detection information received by the reception unit 221, the stimulus determination unit 222 determines the details of the stimulus to be applied to the object body by the implant device 10. As mentioned above, the detection information represents an electrical signal at a predetermined detection part in the object body, detected by the implant device 10. The stimulus determination unit 222 uses this detection information to acquire information regarding the time variation of the physiological signal at the predetermined part in the object body. For example, when the detection information includes information representing one electrical signal, as a result of detection for one time, the stimulus determination unit 222 accumulates and stores the detection information for a plurality of times to acquire information representing the time variation of the electrical signal (physiological signal at a predetermined part in the object body). This information representing the time variation is time-domain information, but based on such time-domain information, an ideal waveform as exemplified in FIG. 7(a) cannot be obtained due to the noises. For example, as can be seen from FIG. 7(b) showing an example sampled at 2000 Hz, only a series of signals representing a general tendency can be obtained. Accordingly, the variation of responses by nerve, etc., to the stimuli cannot be clearly grasped from the time-domain information.

Therefore, as an example, the stimulus determination unit 222 transforms this time-domain information, that is, the information representing the time variation of the object body physiological signal at the predetermined part in the object body, to frequency-domain information. Thanks to this transformation to the frequency-domain information, the variation of responses to the stimuli can be detected comparatively easily. For this transformation to the to the frequency-domain information, widely known method can be adopted such as FFT (Fast Fourier Transform), and the like.

Specifically, FIG. 7(c) shows a waveform obtained by transforming the signals exemplified in FIG. 7(b) to frequency-domain information. FIG. 7(c) is obtained by sequentially transforming parts of the waveform shown in FIG. 7(b), each part having 1.024 seconds (when sampling is performed for 2048 samples, namely, sampling is performed at 2000Hz, the number of samples in 1.024 seconds is 2048), to frequency-domain information by FFT, and FIG. 7(c) shows the frequency-domain information with the signal intensity of 300 Hz to 400 Hz (shows time variation of the signal intensity of the above frequency domain). It can be found that FIG. 7(c) shows an envelope having a waveform pattern similar to the ideal signal (FIG. 7(a)).

Namely, the stimulus determination unit 222 transforms the time variation of the electrical signal to the signal intensity information of every frequency component, by the FFT, etc. Specifically, when the detection information received from the implant device 10 is information representing a detection result of less than the predetermined 2ⁿ times (n being a natural number of 1 or more) of detection of the electric signal, such that the information is transmitted from implant device 10 every time that the electrical signal is detected (that is, in case of the information regarding one-time detection), and the like, the stimulus determination unit 222 accumulates the received information and stored in the storage unit 23 until detection results of 2ⁿ times of detection of the electrical signal are obtained.

Then, the stimulus determination unit 222 treats the stored detection result of 2ⁿ times of detection of the electrical signal as information regarding the time variation of the physiological signal at a predetermined part in the object body, and transforms this information regarding the time variation of the physiological signal to frequency-domain information by the FFT. On the basis of the frequency-domain information obtained by the transformation, the stimulus determination unit 222 determines the details of the stimulus to be applied to the object body by the implant device 10.

For example, the stimulus determination unit 222 determines the type of stimulus with reference to stimulus setting information in which a plurality of mutually different stimulus applying conditions are associated with information representing the details of the stimulus corresponding to each stimulus applying condition. The stimulus setting information is set in advance by a predetermined method, provided from a personal computer (PC), etc., through a cable or wirelessly, and stored in the storage unit 23.

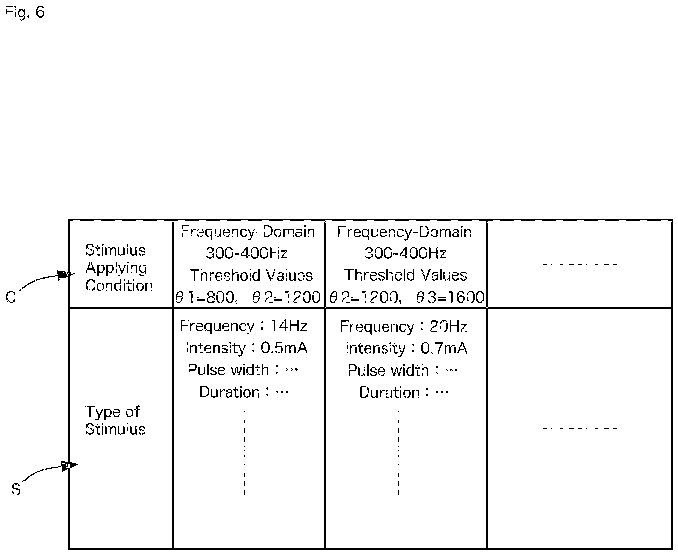

FIG. 6 shows an example of the stimulus setting information. In the stimulus setting information, the stimulus applying condition (C) is associated with the information (S) representing the details of the stimulus. The stimulus applying condition (C) includes, for example, frequency band information and signal intensity information at the frequency band.

Further, the information (S) representing the details of the stimulus includes frequency of the stimulus (pulse signal) to be applied, intensity of the stimulus, pulse width of the stimulus, providing period of the stimulus, and the like.

In the example shown in FIG. 6, for example, a stimulus applying condition that a signal F with a predetermined frequency component (as an example, a signal with 300 Hz to 400 Hz) has an intensity of higher than a predetermined first threshold value θ1 and lower than a second threshold value θ2 (with a proviso that θ2>θ1 is satisfied), is associated with information representing the details of the stimulus that "providing a comparatively weak stimulus (such as, with an amplitude of 0.5 mA) with the frequency f1" (by determining in advance that, for example, f1=14 Hz).

Further, a stimulus applying condition that the above signal F has an intensity of higher than the second threshold value θ2 and lower than the third threshold value θ3 (with a proviso that θ3>θ2 is satisfied), is associated with information representing the details of the stimulus that "providing a slightly strong stimulus (such as, with an amplitude of 0.7 mA) with the frequency f2" (by determining in advance that, for example, f2=20 Hz).

Similarly, a plurality of mutually different stimulus applying conditions are respectively associated with information representing the details of the stimulus to be applied when each of the stimulus applying conditions is satisfied. With this respect, the stimulus applying condition may be a condition relating to one of the threshold values such that the signal F has an intensity of higher than the N-th threshold value θN.

The stimulus determination unit 222 refers to the frequency-domain information obtained by the FFT. When, for example, the stimulus setting information shown in the example of FIG. 6 is defined, the stimulus determination unit 222 refers to the information of the intensity regarding the signal with 300 Hz to 400 Hz in the stimulus setting information. If the intensity of the signal represented by the information is higher than the first threshold value θ1, and lower than the second threshold value θ2, the stimulus applying condition shown in the first column of FIG. 6 is satisfied. Thus, the details of the stimulus defined by the corresponding information, that is, "providing stimulus with amplitude 0.5 mA at frequency f1=14 Hz", is determined as a stimulus to be applied.

Furthermore, when the frequency-domain information obtained by the FFT does not satisfy any one of the stimulus applying conditions included in the stimulus setting information, that is, in the example of FIG. 6, for example, when the signal F with a predetermined frequency component (as an example, a signal with 300 Hz to 400 Hz) has an intensity of lower than the predetermined first threshold value θ1, the stimulus determination unit 222 can determine that "no stimulus is to be applied", or determine to apply a stimulus previously determined as a default.

The stimulus determination unit 222 outputs the information representing the details of the stimulus determined by the method exemplified above, to the instruction transmission unit 223.

The instruction transmission unit 223 transmits the stimulation instruction representing the details of the stimulus determined by the stimulus determination unit 222, to the implant device 10.

The stimulus setting information used here may be defined on the basis of the detection result of the electrical signal representing the physiological signal, which is acquired by the implant device 10.

Specifically, according to an example of the present aspect, the controller device 20, or an information processing device such as a computer communicably connected to the implant device 10 acquires information representing the time variation of the electrical signal which represents the physiological signal of the object body, the electrical signal being obtained by the implant device 10 implanted in the object body at a predetermined time interval (for example, every 1/2000 second) within a predetermined period (for example, 24 hours). Then, the information processing device such as the controller device 20, etc., transforms the information obtained from the implant device 10 to the signal intensity information for each frequency component using a method such as FFT, etc.

Then, the information processing device extracts signal intensity information of a predetermined frequency (for example, 300 Hz) from the signal intensity information for each frequency component obtained by the transformation, and classifies the extracted signal intensity information into a plurality of classes by a clustering process. For this clustering process, widely known methods such as a k-means method can be adopted.

Here, for example, when the signal intensity information is classified into N classes (N being 3 or more), threshold values θ1, θ2, ..., separating the respective classes are obtained. Each stimulus applying condition is determined to include a set of threshold values, each set including two threshold values selected from the threshold value θ1, θ2, ... (the threshold-value sets are selected so that the ranges defined by the respective threshold-value sets do not overlap with each other).

Further, the implant device 10 which obtains the detection result which is a base of the signal classified by the clustering process can be implanted in a specific object body (individual body) wearing the controller device 20 in which the threshold value is set, or can be implanted in another individual body in the same kind of the relevant individual body (for example, in case that the individual body is a human being, not the individual human being himself/herself, but another human being).

In addition, in the above explanation, the stimulus applying condition is set by comparing the signal intensity information for each frequency component with the threshold value. However, the information processing device such as the controller device 20, etc. can determine the stimulus applying condition using the signal intensity information for each frequency component obtained in the past and referring to the time variation of the signal intensity information for each frequency component (for example, a magnitude of the change in signal intensity per unit time). Further, the controller device 20, etc. can refer to the signal intensity information for each frequency component in the past obtained after the stimulus was actually applied, and determine the details of the stimulus to be applied for setting the stimulus setting information.

In addition, the stimulus setting information can be set by a doctor, etc., with reference to or regardless of the threshold value, etc., determined by the above clustering method, etc.

### [Operation Example]

Next, operations of the stimulus application system 1 according to the present aspect will be explained. In the following example, the implant device 10 is implanted in the object body, i.e., the body of a human being, and electrodes for providing stimuli are arranged at the stimulation parts and the detection parts used for Sacral Neuro Modulation.

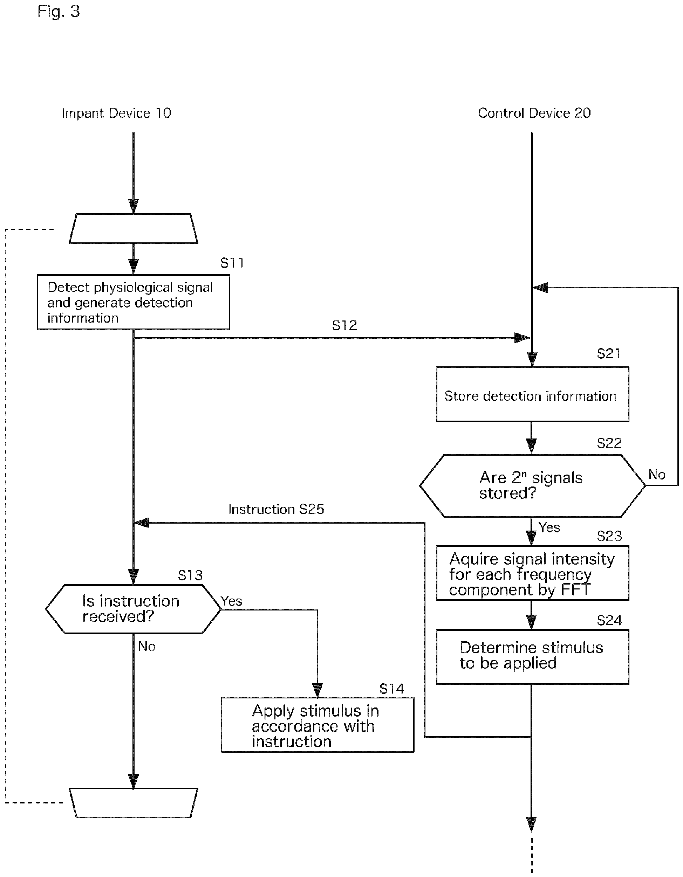

The implant device 10 implanted in the human body executes the process exemplified in FIG. 3 in, for example, every 30 minutes. In this process, first, the implant device 10 detects the electrical signals representing the physiological signals in this human body by the electrodes arranged in the detection parts, and generates detection information representing the detected electrical signals (S11) .

The implant device 10 transmits the generated detection information to the controller device 20 arranged outside of the human body, a predetermined time (for example, every time that the detection is performed) (S12). Then, the implant device 10 examines whether or not an instruction is received from the controller device 20 within a predetermined time (S13). If no instruction is received (S13: No), the process returns to Step S11, and is continued.

On the other hand, in Step S12, the controller device 20 receives and stores the detection information transmitted by the implant device 10 (S21).

The controller device 20 repeatedly executes Step S21 until the electrical signal detection results of a predetermined 2ⁿ times (n being a natural number of 1 or more, for example, n=11) of detection are obtained, and accumulates and stores 2ⁿ electrical signals (S22). Then, when detection information representing the 2ⁿ electrical signals is accumulated, the controller device 20 transforms the information represented by the 2ⁿ electrical signals and representing time variation of the physiological signal at a predetermined part in the object body, to signal intensity information for each frequency component by the FFT (S23).

The controller device 20 refers to the signal intensity information for each frequency component obtained in Step S23 and predetermined stimulus setting information, and determines the type of stimulus to be applied (S24). In this determination process, as already mentioned above, for example, the controller device 20 refers to the signal intensity information for each frequency component, and determines whether or not the intensity of the signal F satisfies either one of the stimulus applying conditions included in the stimulus setting information exemplified in FIG. 6:
(a) The intensity of the signal F of a predetermined frequency component (for example, 300 Hz to 400 Hz, same in below) is higher than a first threshold value θ1, and lower than a second threshold value θ2 (with a proviso that θ2>θ1 is satisfied);
(b) The intensity of the signal F of a predetermined frequency component is higher than the second threshold value θ2, and lower than a third threshold value θ3 (with a proviso that θ3>θ2 is satisfied) ....

Then, for example, if none of the stimulus applying conditions included in the stimulus setting information is satisfied, such that the signal intensity information for each frequency component which is referred to is lower than the predetermined first threshold value θ1, the controller device 20 determines the stimulus to be applied as "no stimulus is to be applied".

Further, if the signal intensity information for each frequency component which is referred to satisfies the stimulus applying condition (a), i.e., the signal intensity at a predetermined frequency component is higher than the first threshold value θ1, and lower than a second threshold value θ2 (with a proviso that θ2>θ1 is satisfied), the controller device 20 uses the information representing the details of the stimulus which is set in association with the stimulus applying condition (a), and determines the stimulus to be applied as "stimulus with a frequency of 14 Hz, and an amplitude of 0.5 mA".

Further, if the signal intensity information for each frequency component which is referred to satisfies the stimulus applying condition (b), i.e., the signal intensity at a predetermined frequency component is higher than the second threshold value θ2, and lower than a third threshold value θ3 (with a proviso that θ3>θ2 is satisfied), the controller device 20 uses the information representing the details of the stimulus which is set in association with the stimulus applying condition (b), and determines the stimulus to be applied as "stimulus with a frequency of 20 Hz, and an amplitude of 0.7 mA".

The controller device 20 transmits an instruction representing the details of the stimulus determined in Step S24, to the implant device 10 (S25).

If the implant device 10 receives the instruction from the controller device 20 in Step S13 (S13: Yes), the implant device 10 determines parameters, such as a frequency, an amplitude, etc., of the electrical signal as a stimulus to be applied to the human body in which the implant device 10 is implanted, and controls a current to be applied to the stimulation part through the electrode so that the stimulus of the electrical signal with the determined parameters is to be applied (S14).

The stimulus application system 1 according to the present aspect repeats the operations from Step S11 to Step S14, and the operations from Step S21 to Step S25.

Accordingly, according to an example of the present aspect, the stimulus to be applied is varied in accordance with the status of the object human body, etc., and thus, a stimulus suitable for the status of the object can be applied.

### [Synthesis of Frequency-Domain Information]

Further, the controller device 20 does not have to determine the details of the stimulus by directly using the signal intensity information for each frequency component based on the detection information representing 2ⁿ electrical signals. Instead, the controller device 20 may repeatedly execute Step S21 to Step S23 of the process exemplified in FIG. 3 to obtain the signal intensity information for each frequency component for a plurality of times.

In this case, the controller device 20 may hold frequency-domain information obtained by transforming, by the FFT method, each of the information acquired for a predetermined plurality of times in the past, regarding the time variation of the physiological signal at a predetermined part in the object body (information based on the detection information representing 2ⁿ electrical signals). Here, the information based on the detection information representing 2ⁿ electrical signals used for each execution of the FFT may not mutually overlap, or may include overlapping information. For example, one way may be using the detection information representing the detection results of first to 2ⁿ times of detection in the initial FFT, and using the detection information representing the detection results of 2ⁿ+1 to 2×2ⁿ times of detection in the next FFT, and so on. Another way, wherein overlapping is allowed, may be using the detection information representing the detection results of first to 2ⁿ times of detection in the initial FFT, and using the detection information representing the detection results of 2ⁿ⁻¹+1 to 2ⁿ⁻¹+2ⁿ times of detection in the next FFT, and so on.

Then, the controller device 20 may synthesize the held frequency-domain information of the past plurality of times, and determine the type of stimulus to be applied to the object body by the implant device 10, with reference to the synthesized frequency-domain information and the stimulus setting information.

As a method for the synthesis, various methods for obtaining a predetermined statistical value of the signal intensity with respect to each corresponding frequency component can be adopted, such as a method for accumulating signal intensities of respectively corresponding frequency components, a method for accumulating signal intensities and then dividing the accumulation result by the number of accumulated signal intensities (namely, obtaining an arithmetic average of the signal intensity for each frequency component), and the like.

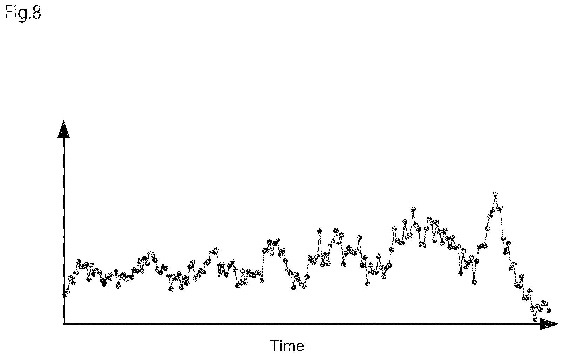

By way of example, FIG. 8 shows a result obtained by sequentially accumulating the results of 10-times of the FTT exemplified in FIG. 7(c). It is found that the waveform shown in FIG. 8 is more similar to the ideal waveform shown in FIG. 7(a) than the waveform in FIG. 7(c) is. Accordingly, when the statistical value, such as an average, etc. is calculated after the transformation to the frequency-domain information, changes in the physiological signal can be more clearly analyzed without being influenced by a temporary noise, etc.

The controller device 20 refers to the synthesis result of the signal intensity for the frequency component (synthesized frequency-domain information), and the stimulus setting information, and uses the stimulus setting information, for example, shown in FIG. 6. When the stimulus applying condition that the arithmetic average of signal intensities obtained for a plurality of times at a predetermined frequency component (300 Hz to 400 Hz) is higher than the first threshold value θ1, and lower than the second threshold value θ2 (with a proviso that θ2>θ1 is satisfied), is satisfied, the controller device 20 uses the information representing the details of the stimulus which is set corresponding to this stimulus applying condition, and determines the stimulus to be applied as "stimulus with a frequency of 14 Hz, and an amplitude of 0.5 mA". Also, when the stimulus applying condition that the arithmetic average of signal intensities obtained for a plurality of times at a predetermined frequency component (300 Hz to 400 Hz) is higher than the second threshold value θ2, and lower than the third threshold value θ3 (with a proviso that θ3>θ2 is satisfied), is satisfied, the controller device 20 uses the information representing the details of the stimulus which is set corresponding to this stimulus applying condition, and determines the stimulus to be applied as "stimulus with a frequency of 20 Hz, and an amplitude of 0.7 mA".

According to this example of the present aspect, transformation to the frequency-domain information is performed, and thereafter, a statistical value, such as an average, is calculated. Thereby, if a temporal noise contamination of the signal occurs, there are no influence therefrom, and the stimulus can be applied more appropriately.

### [Embodiments of Stimulus to be applied]

Regarding Step S24 of the process in the example shown in FIG. 3, as an embodiment of the stimulus to be applied on the basis of the signal intensity information for each frequency component, an example of controlling the frequency and the amplitude of the periodic electrical stimulus is described above. However, as is explained above, this is merely an example, and in Step S24, a pulse width of the stimulus applied, time of applying the stimulus, duration of the stimulus, etc., may be further determined. These embodiments of the stimulus determined on the basis of the signal intensity for each frequency component can be determined experimentally.

According to an example of the present aspect, a server device (not shown in the drawing) which is communicably connected to the controller device 20 through a network can determine the details of the stimulus to be applied, by using the detection information or the signal intensity information for each frequency component obtained on the basis of the detection information. The process by the server device may be the same as the process by the above-mentioned stimulus determination unit 222.

In this example, instead of the above-mentioned process, and as a process of the stimulus determination unit 222, the controller device 20 transmits the detection information received by the reception unit 221, or the signal intensity information for each frequency component obtained by transforming the received detection information by FFT, etc., to the server device, receives the information representing the details of the stimulus from the server device, and outputs the received information to the instruction transmission unit 223.

### [Stimulation Trial]

According to an example of the present aspect, the controller device 20 can transmit stimulation instructions (trial stimulation instructions) representing a plurality of prospective stimuli having mutually different stimulus details, at each predetermined time, to the implant device 10, and after the transmission of the trial stimulation instructions, the controller device 20 can evaluate the effect of the transmitted trial stimulation instructions using the detection information received from the implant device 10. According to this example, the controller device 20 provides the evaluation results of each of the stimulation instructions respectively corresponding to the plurality of prospective stimuli to a predetermined process relating to the determination of the stimulation instruction.

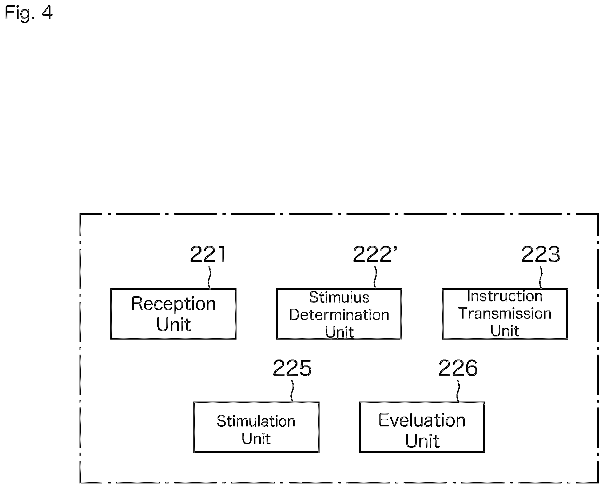

Specifically, as exemplified in FIG. 4, the control unit 22 of the controller device 20 according to this example functionally includes a reception unit 221, a stimulus determination unit 222' , an instruction transmission unit 223, trial stimulation unit 225, and an evaluation unit 226. Here, the units operating in the same way as those in the Example of FIG. 2 are assigned with the same numerals, and details explanation therefor is omitted.

In the present example, the trial stimulation unit 225 transmits stimulation instructions respectively representing a plurality of prospective stimuli having mutually different stimulus details, at each predetermined time, to the implant device 10. For example, this trial stimulation unit 225 tries the stimulus with a predetermined stimulus pattern, as exemplified below.

Namely, according to an example of the present aspect, the trial stimulation unit 225 receives settings regarding the plurality of prospective stimuli having mutually different stimulus details and the time to transmit the stimulation instructions respectively representing the prospective stimuli, from a personal computer, etc., communicably connected to the trial stimulation unit 225 through the communication unit 24, by the operations by a doctor, etc.

Specific settings are as below:
(1) output a stimulation instruction representing a prospective stimulus of 14 Hz, 1.0 mA, and a duration of 10 seconds,
(2) standby for 30 seconds,
(3) output a stimulation instruction representing a prospective stimulus of 15 Hz, 1.0 mA, and a duration of 10 seconds,
(4) standby for 30 seconds,
(5) output a stimulation instruction representing a prospective stimulus of 16 Hz, 1.0 mA, and a duration of 10 seconds,
(6) standby for 30 seconds, ....
As explained below, during the trial stimulation unit 225 is on standby after the output of the stimulation instruction, the implant device 10 performs electrical stimulation in accordance with the stimulation instruction, and thereafter detects the electrical signal by the electrode arranged at the detection part, as a physiological signal in the object body, and obtains detection information representing the time variation of the electrical signal.

In the example here, the details of the mutually different stimuli are mutually different frequencies of the periodic electrical stimuli. However, the present aspect is not limited thereto. For example, at least one thing may be made mutually different among, for example: amplitudes of periodic electrical stimuli, durations of the stimuli, pulse widths of the stimuli, and frequencies of the stimuli. Further, in case of another embodiment of the stimulus, for example, in case that the stimulus circuit unit 13 has a plurality of (three or more) electrodes, or in case that a plurality of the stimulus circuit units 13 are provided, namely, there a plurality of prospective stimulation parts, the stimulation part to be stimulated may be made different. As far as mutually different stimuli can be applied to the object body, various modified examples can be used.

In accordance with the received settings, the trial stimulation unit 225 outputs a stimulation instruction representing (1) prospective stimulus of 14 Hz, 1.0 mA, and a duration of 10 seconds, to the implant device 10, and (2) is on standby for 30 seconds. Further, the trial stimulation unit 225 outputs the information representing the details of the stimulation instruction output to the implant device 10, to the evaluation unit 226.

At this time, the implant device 10 receives the stimulation instruction from the controller device 20, and in accordance with the stimulation instruction, determines a parameter such as a frequency, an amplitude, etc., of the electrical signal as a stimulus to be applied to the human body in which the implant device 10. Here, in accordance with the instruction, an electrical stimulus with 14 Hz, 1.0 mA, and a duration of 10 seconds is applied to the stimulation part of the object body.

Then, while the trial stimulation unit 225 is on standby for 30 seconds, the implant device 10 detects the electrical signal as a physiological signal in the object body, by the electrode arranged at the detection part, and stores the detection information representing the time variation thereof. At a predetermined time thereafter (here, before the trial stimulation unit 225 outputs the next trial instruction), the implant device 10 transmits the stored detection information to the controller device 20. The detection information is to be processed by the evaluation unit 226 explained below.

As above, the trial stimulation unit 225 (2) is on standby for 30 seconds, thereafter, (3) outputs a stimulation instruction representing a prospective stimulus of 15 Hz, 1.0 mA, and a duration of 10 seconds, and then, (4) is on standby for 30 seconds. Further, the trial stimulation unit 225 outputs the information representing the details of the stimulation instruction output to the implant device 10, to the evaluation unit 226.

Also at this time, the implant device 10 receives the stimulation instruction from the controller device 20, and in accordance with the stimulation instruction, applies the electrical stimulus with 5 Hz, 1.0 mA, and a duration of 10 seconds to the stimulation part in the object body. Then, while the trial stimulation unit 225 is on standby for 30 seconds, the implant device 10 detects the electrical signal as a physiological signal in the object body, by the electrode arranged at the detection part, and stores the detection information representing the time variation thereof. At a predetermined time thereafter (here, as mentioned above, before the trial stimulation unit 225 outputs the next stimulation instruction), the implant device 10 transmits the stored detection information to the controller device 20.

Hereinafter, the trial stimulation unit 225 repeats operations of transmitting a stimulation instruction representing a prospective stimulus to the implant device 10 in accordance with the setting, and standing-by for a predetermined time. Every time that the implant device 10 receives the stimulation instruction representing a prospective stimulus, the implant device 10 applies an electrical stimulus in accordance with the stimulation instruction to the stimulation part in the object body. Thereafter, during the period that the trial stimulation unit 225 is on standby, the implant device 10 detects the electrical signal as a physiological signal in the object body, obtains detection information, and thereafter, transmits the detection information to the controller device 20.

Note that the operation (and the setting thereof) of the trial stimulation unit 225 explained above is only an example. If another operation is set, the trial stimulation unit 225 operates in accordance with the relevant setting. For example, unlike the above, the trial stimulation unit 225 does not standby for a predetermined time, and may determine the time for outputting the next stimulation instruction as below.

Namely, according to an example of the trial stimulation unit 225, the trial stimulation unit 225 stands by after the stimulation instruction, and if the reception unit 221 receives the detection information during the standby period, the trial stimulation unit 225 determines whether or not the next stimulation instruction is to be output, on the basis of the received detection information. Then, if the trial stimulation unit 225 determines to output the next stimulation instruction, in accordance with the setting, the trial stimulation unit 225 outputs a stimulation instruction representing a prospective stimulus which is to be output next.

As a specific example, in this case, the trial stimulation unit 225 transforms the detection information received by the reception unit 221 to signal intensity information for each frequency component (frequency-domain information) by the FFT, etc. Then, the trial stimulation unit 225 refers to the signal intensity information, and determines to output a stimulation instruction of a corresponding prospective stimulus when, for example, the intensity of a signal F with a predetermined frequency component (by way of example, a signal with 300 Hz to 400 Hz) is higher than the predetermined threshold value θ.

As in the example mentioned above, the threshold value used here can be obtained by a clustering process and the like, on the basis of the plurality of times of detection information obtained during a predetermined period.

According to this example of the present aspect, the trial stimulation unit 225 operates as follows. The trial stimulation unit 225 is on standby until the intensity of the signal F for the predetermined frequency component in the frequency-domain information obtained by transforming the detection information received by the reception unit 221, exceeds the predetermined threshold value θ, and outputs (1) stimulation instruction representing a prospective stimulus with 14 Hz, 1.0 mA, and a duration of 10 seconds. Thereafter, the trial stimulation unit 225 awaits until the intensity of the signal F for the predetermined frequency component in the frequency-domain information obtained by transforming the detection information received by the reception unit 221, exceeds the predetermined threshold value θ again, and outputs (3) stimulation instruction representing a prospective stimulus with 15 Hz, 1.0 mA, and a duration of 10 seconds.

Further, instead of the operations that the trial stimulation unit 225 is on standby until a set time is passed or until the time determined on the basis of the detection information received by the reception unit 22, and outputs the stimulation instruction representing the prospective stimulus; the trial stimulation unit 225 can control the time to output the stimulation instruction representing a prospective stimulus, by a combination of the time when the set time has passed and the time determined on the basis of the detection information received by the reception unit 221, such that if the predetermined time has not passed after the output of the stimulation instruction representing the previous prospective stimulus at the time when the trial stimulation unit 225 determines to output the stimulation instruction representing the prospective stimulus on the basis of the detection information received by the reception unit 221, the trial stimulation unit 225 stays on standby for the predetermined time.

After the trial stimulation unit 225 transmits the stimulation instruction, namely, after the evaluation unit 226 receives information representing the details of the stimulation instruction transmitted from the trial stimulation unit 225, the evaluation unit 226 receives the detection information from the implant device 10. Then, on the basis of the detection information, the evaluation unit 226 evaluates whether or not there is an effect of the stimulation instruction transmitted from the trial stimulation unit 225, or the degree of the effect.

According to an example of the present aspect, the evaluation unit 226 transforms the detection information which is the time-domain information to the frequency-domain information using the FFT. Then, the evaluation unit 226 evaluates a degree of the effect of the stimulation instruction transmitted by the trial stimulation unit 225 at the intensity of the signal F with a predetermined frequency component (by way of example, a signal with 300 Hz). For example, regarding the detection information corresponding to the plurality of mutually different stimulation instructions transmitted by the trial stimulation unit 225, the evaluation unit 226 compares the intensities of the signal F with the predetermined frequency component (by way of example, a signal with 300 Hz), and specifies the stimulation instruction corresponding to the detection information having the smallest intensity. Then, the evaluation unit 226 treats the specified stimulation instruction as the most effective stimulation instruction, and outputs the corresponding stimulation instruction details to the stimulus determination unit 222'.

Here, an example in which intensities of the signal F with the predetermined frequency component (by way of example, a signal with 300 Hz) are compared regarding the detection information corresponding to the plurality of mutually different stimulation instructions transmitted by the trial stimulation unit 225, and the stimulation instruction corresponding to the detection information having the smallest intensity is specified. However, the present disclosure is not limited to this example. The evaluation unit 226 can examine the time variation of the intensity of the signal F with the predetermined frequency component (by way of example, a signal with 300 Hz) regarding the detection information corresponding to the plurality of mutually different stimulation instructions transmitted by the trial stimulation unit 225, and determine the most effective stimulation instruction based on the time variation (for example, the magnitude of the decreasing rate per unit time, etc.)

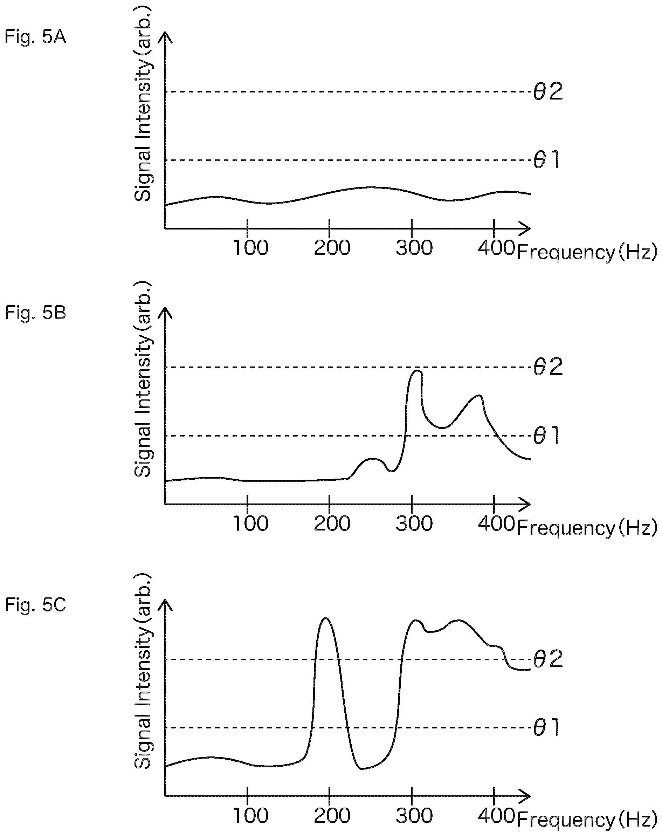

Same as the above example, the trial stimulation unit 225 performs the following, respectively:
(1) output a stimulation instruction representing a prospective stimulus with 14 Hz, 1.0 mA, and a duration of 10 seconds,
(3) output a stimulation instruction representing a prospective stimulus with 15 Hz, 1.0 mA, and a duration of 10 seconds,
(5) output a stimulation instruction representing a prospective stimulus with 16 Hz, 1.0 mA, and a duration of 10 seconds,
and thereafter, a detection signal is obtained. FIG. 5 shows an example of the frequency-domain information obtained by transforming the obtained detection signal by the FFT. In FIG. 5, the horizontal axis represents a frequency (Hz), and the vertical axis represents a signal intensity (arbitrary unit).

As exemplified in FIG. 5, the detection signal corresponding to (a) prospective stimulus with 14 Hz, 1.0 mA, and a duration of 10 seconds has a smaller intensity of the signal F with the predetermined frequency component (by way of example, a signal with 300 Hz), compared to the detection signal corresponding to other cases, i.e., (b) prospective stimulus with 15 Hz, 1.0 mA, and a duration of 10 seconds, and (c) prospective stimulus with 16 Hz, 1.0 mA, and a duration of 10 seconds. At this time, the evaluation unit 226 determines that the case that the signal F has the smallest intensity, i.e., (a) prospective stimulus with 14 Hz, 1.0 mA, and a duration of 10 seconds, as the most effective prospective stimulus.

Such operations of the evaluation unit 226 can be performed by using widely known processes, such as a sorting process, and thus, further detailed explanation therefor is omitted here.

Further, according to the determination based on the intensities of the above signals F corresponding to the mutually different stimulation instructions, if the signals F corresponding to the stimulation instructions of the prospective stimuli determined as the most effective have substantially the same intensity, among the mutually different stimulation instructions, a stimulation instruction with a lower (or higher) frequency (or a stimulation instruction with a smaller (or higher) amplitude (stimulus intensity)) may be determined as the most effective stimulation instruction, and the information representing the details thereof may be output. In case that the signals F corresponding to the mutually different stimulation instructions have substantially the same intensity, determination as to which stimulation instruction should be selected may be performed by, for example, selecting a stimulation instruction by which a burden on the object body is comparatively small, and the like. The stimulus determination unit 222' performs the operations same as the above-explained stimulus determination unit 222, and in addition, receives the input of information output from the evaluation unit 226 and representing the details of the most effective stimulation instruction, and stores the information representing the details of the relevant stimulation instruction.

Thereafter, same as the operations of the above-mentioned stimulus determination unit 222, the stimulus determination unit 222' transforms the detection information received by the reception unit 221 by the FFT 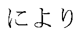 frequency-domain information to obtain the intensity of the signal F with the predetermined frequency component (by way of example, a signal with 300 Hz). Then, if the obtained intensity is lower than the predetermined first threshold value θ1, the stimulus determination unit 222' can determine that "no stimulus is to be applied". Whereas, if the obtained intensity of the signal F is higher than the first threshold value θ1, the stimulus determination unit 222' outputs the information representing the details of the stimulus on the basis of the stored stimulation instruction details to the instruction transmission unit 223.

Further, the stimulus determination unit 222' of this example can modify the stored stimulation instruction details, on the basis of the detection information, that is, for example, on the basis of the intensity of the signal F with the predetermined frequency component (by way of example, a signal with 300 Hz) obtained by transforming the detection information. For example, if the signal F has an intensity of higher than the first threshold value θ1, and lower than the second threshold value θ2 (with a proviso that θ2>θ1 is satisfied), the stimulus determination unit 222' can modify the information representing the amplitude in the stored stimulation instruction details, and output information representing stimulus details on the basis of the modified stimulation instruction details, to the instruction transmission unit 223. Also, if the signal F has an intensity of higher than both the first threshold value θ1 and the second threshold value θ2, the stimulus determination unit 222' can output information representing stimulus details on the basis of the stimulation instruction details without modifying the stored stimulation instruction details, to the instruction transmission unit 223.

In this Example, in case that the stored stimulation instruction details is "14 Hz, 1.0 mA, and a duration of 10 seconds", if the signal F has an intensity of higher than the first threshold value θ1, and lower than the second threshold value θ2 (with the proviso that θ2>θ1 is satisfied), the stimulus determination unit 222' modifies the stored stimulation instruction details to "14 Hz, 0.5 mA, and a duration of 10 seconds", and outputs information representing stimulus details on the basis of the modified stimulation instruction details, to the instruction transmission unit 223. On the other hand, if the signal F has an intensity larger than both the first threshold value θ1 and the second threshold value θ2, the stimulus determination unit 222' does not modify the stored stimulation instruction details, and outputs information representing stimulus details on the basis of the stored stimulation instruction details, i.e., "14 Hz, 1.0 mA, and a duration of 10 seconds", to the instruction transmission unit 223.

The modification explained here is merely an example. The stimulus determination unit 222' can modify at least one of the amplitude of periodic electrical stimulus, the duration of the stimulus, the pulse width of the stimulus, and the frequency of the stimulus, on the basis of the detection information.

Also in this example, a server device (not shown in the drawing) which is communicably connected to the controller device 20 through a network can determine the details of the stimulus to be applied, by performing processes corresponding to the processes by the above-mentioned stimulus determination unit 222'.

In this case, instead of the above-mentioned process, and as a process of the stimulus determination unit 222', the controller device 20 transmits the detection information received by the reception unit 221, or the signal intensity information for each frequency component obtained by transforming the received detection information by FFT, etc., and information output by the evaluation unit 226 and representing the details of the most effective stimulation instruction, to the server device, receives the information representing the details of the stimulus to be applied from the server device, and outputs the received information to the instruction transmission unit 223.

### [Example of Processing at Implant Device Side]

According to the present aspect, the implant device 10 can execute the processes executed by the controller device 20 in the object body. According to this example, the implant device 10 detects the electrical signal representing the physiological signal in the human body by the electrode arranged at the detection part, and generates the detection information representing the detected electrical signal.

Then, the implant device 10 according to this example accumulates and stores the generated detection information in the memory of the processor unit 15. The implant device 10 repeats the detection of the electrical signal, the generation of the detection information, and the storing, until a result of a predetermined 2ⁿ times (n being a natural number of 1 or more, for example, n=11) of electrical signal detection is obtained. When 2ⁿ electrical signals are accumulated in the processor unit 15, the implant device 10 transforms the information representing the physiological signal at a predetermined part in the object body, which is represented by the 2ⁿ electrical signals, to the signal intensity information for each frequency component, by, for example, FFT.

The implant device 10 refers to the signal intensity information for each frequency component obtained by this process and the predetermined stimulus setting information, and determines the details of the stimulus to be applied. According to this example of the present aspect, the implant device 10 stores the stimulus setting information set in advance (same as the one exemplified in FIG. 6). The implant device 10 refers to, for example, and determines whether or not the intensity of the signal F satisfies the any one of the stimulus applying conditions included in the stored stimulus setting information.

Here, if none of the stimulus applying conditions included in the stimulus setting information stored in the implant device 10 is satisfied, the implant device 10 may determine the stimulus to be applied as "no stimulus is to be applied".

If the implant device 10 determines that the signal intensity information for each frequency component that is referred to, satisfies any one of the stimulus applying conditions included in the stored stimulus setting information, the implant device 10 acquires information of the stimulus details associated with the stimulus applying condition satisfied by the signal intensity information for each frequency component that is referred to.

On the basis of the acquired stimulus details, the implant device 10 determines parameters, such as a frequency, an amplitude, etc., of the electrical signal as a stimulus to be applied to the object body, i.e., the human body in which the implant device 10 is implanted, and controls a current to be applied to the stimulation part through the electrode so that the stimulus of the electrical signal with the determined parameters is to be applied.

This operation of the implant device 10 may be performed when, for example, the time period during which the implant device 10 is incommunicable with the controller device 20 is longer than a predetermined time period. Further, the stimulus setting information used by the implant device 10 in the above operation may be the same as or different from the stimulus setting information used by the controller device 20. For example, the stimulus setting information used by the implant device 10 may be a sub-set of the stimulus setting information used by the controller device 20 (including a part of the stimulus applying condition and information representing the stimulus details corresponding thereto).

Furthermore, when the implant device 10 performs a process such as the FFT, etc., the implant device 10 does not have to determine the details of the stimulus by directly using the signal intensity information for each frequency component based on the detection information representing 2ⁿ electrical signals. Instead, the implant device 10 may repeatedly execute the detection of the electrical signal, the generation and storing of the detection information, and the process to obtain the signal intensity information for each frequency component, to thereby obtain the signal intensity information for each frequency component (frequency-domain information) for a plurality of times.

In this case, the implant device 10 may hold frequency-domain information obtained by transforming, by the FFT method, each of the information acquired for a predetermined plurality of times in the past, regarding the time variation of the physiological signal at a predetermined part in the object body (information based on the detection information representing 2ⁿ electrical signals). Here, the information based on the detection information representing 2ⁿ electrical signals used for each execution of the FFT may not mutually overlap, or may include overlapping information. For example, one way may be using the detection information representing the detection results of first to 2ⁿ times of detection in the initial FFT, and using the detection information representing the detection results of 2ⁿ+1 to 2×2ⁿ times of detection in the next FFT, and so on. Another way, wherein overlapping is allowed, may be using the detection information representing the detection results of first to 2ⁿ times of detection in the initial FFT, and using the detection information representing the detection results of 2ⁿ⁻¹+1 to 2ⁿ⁻¹+2ⁿ times of detection in the next FFT, and so on.

Then, the implant device 10 may synthesize the held frequency-domain information of the past plurality of times, and determine the type of stimulus to be applied to the object body, with reference to the synthesized frequency-domain information and the stimulus setting information.

Also in this example, as a method for the synthesizing the frequency-domain information, various methods for obtaining a predetermined statistical value of the signal intensity with respect to each corresponding frequency component can be adopted, such as a method for accumulating signal intensities of respectively corresponding frequency components, a method for accumulating signal intensities and then dividing the accumulation result by the number of accumulated signal intensities (namely, obtaining an arithmetic average of the signal intensity for each frequency component), and the like.

### [Example Using Machine Learning]

In the above explanation, for example, the details of the stimulation instruction output by the stimulus determination unit 222 is determined in advance. However, aspects of the present disclosure are not limited thereto.

Regarding the species (species such as human, pig, etc.) same as the species of the object body, a machine-learning model may be generated, in advance and by experiments, to perform machine learning of the relationship between the frequency component of the detection information (frequency-domain information after the transformation by the FFT) before the stimulus is applied (status before the stimulation), and the details (frequency, amplitude, etc.) of the stimulus which are determined as effective to improve the status before the stimulation by, for example, a doctor, and the controller device 20 may execute a process using the machine-leaning model.

In this case, the stimulus determination unit 222 receives detection information, and transforms the time variation of the electrical signal to the signal intensity information for each frequency component by FFT, etc. Thereafter, in the process of Step S14 exemplified in FIG. 3, the above-mentioned information after the transformation is input to the machine-learning model, and the details of the stimulus to be applied is determined as the output therefrom.

The process by the stimulus determination unit 222 in this example can be also performed by the server device communicably connected to the controller device 20 as in the above-mentioned examples.

### [Real-Time Monitoring Function]

According to an example of the present aspect, the implant device 10 sequentially transmits the detection information representing the electrical signal of the detection result. Therefore, the electrical signal represented by the detection information can be further transferred at the controller device 20 side, to the external personal computer, smartphone, etc., so as to be displayed or analyzed. Thereby, real-time monitoring of the physiological signal can be performed.

### [Application Example]

The stimulus application system 1 according to the present aspect can be used for suppressing the symptom of, for example, overactive bladder (OAB), Fecal incontinence, Pain management, epilepsy, Alzheimer, and others.

### [Effect of Aspect]

As explained above, according to an aspect of the present disclosure, the implant device 10 is implanted in the object body, and the controller device 20 is attached on the outside of the object body to be wirelessly communicable with the implant device 10.

The controller device 20 sequentially and wirelessly receives the detection information from the implant device 10 implanted in the object body, the detection information being obtained by the implant device 10 by detecting the electrical signal representing the physiological signal at the stimulation part in the object body.

At this time, for the fast Fourier transformation (FFT) to be performed thereafter, (1) the implant device 10 accumulates the detection information representing the detection results of a predetermined number-th power of 2 (for example, 2048) pieces of the electrical signals, and thereafter, transmits the detection information of the number-th power of 2 times of detection to the controller device 20; or (2) the implant device 10 transmits the detection information representing the detection result of less than a predetermined number-th power of 2 (for example, 2048) pieces of the electrical signals, corresponding to, for example, each execution of detection, and the controller device 20 accumulates and stores the detection information of the predetermined number-th power of 2 times of the detection.

In case of the latter example (2), the implant device 10 may transmit the detection result every time that the detection is executed, without storing the detection information. Thereby, for example, in case that the detection is executed once in 1/2000 seconds, (namely, at a sample rate of 2000 Hz), the implant device 10 does not have to be provided with a high speed and high power consumption memory for shortening the time required for storing the information, or a large capacity memory for storing a large amount of information.

Further, since the detection information is sequentially transmitted, for example, if a necessary and sufficient number of detection results have been experimentally obtained, for obtaining necessary frequency-domain information, details of the necessary stimulus can be determined from a smaller number of detection results. For example, if a necessary and sufficient result can be obtained from results of 2048 times of detection, the time for obtaining the results of 2048 times of detection is merely approximately one second (in case that the sample rate is 2000 Hz). Therefore, even if the following process is performed, the time required from the detection to the application of stimulus can be shortened.

The controller device 20 performs the FFT calculation for the obtained information of the power of 2 times of detection (information representing the time variation of the physiological signal detected for the power of 2 times
at a predetermined time interval), and acquires the signal intensity of the physiological signal for each frequency domain.

At this time the number of actually detected data points is the power of 2, and thus, the FFT calculation can be performed without adding data for the shortfall by padding, etc., the actual detection result can be reflected in the FFT calculation result, and the calculation efficiency can be increased.

Further, the controller device 20 can
(3) repeatedly execute the above processes of acquiring the detection information of the power of 2 times of detection, and performing the FFT calculation, for a plurality of times, and synthesize the results of the respective FFT calculations. The synthesis can be performed by accumulation, calculation of average, etc., Thereby the accuracy of the FFT calculation result can be improved.

Further, according to an example of the present aspect, the stimulus setting information is set in the controller device 20 in advance, the stimulus setting information including a plurality of mutually different stimulus applying conditions associated with the information representing the details of stimulus corresponding to the stimulus applying conditions, respectively.

With respect to the details of the stimuli to be applied, which are associated with the different stimulus applying conditions, at least one of the following is mutually different:
- amplitude of the stimulus,
- duration of the stimulus,
- pulse width of the stimulus, and
- a frequency of the stimulus

Then, the controller device 20 refers to the result of the FFT calculation (when synthesized, the result of the synthesis), and the plurality of stimulus applying conditions included in the stimulus setting information, and searches for a stimulus applying condition satisfied by the result of the FFT calculation.

If the stimulus applying condition satisfied by the FFT calculation is not included in the stimulus setting information, the controller device 20 may determine that no stimulus is to be applied. On the other hand, if the stimulus applying condition satisfied by the FFT calculation result is retrieved, the controller device 20 acquires the stimulus details information associated with the retrieved stimulus applying condition, and wirelessly transmits the instruction instructing to apply the stimulus represented in the information, to the implant device 20.

Upon receiving the instruction to apply the stimulus, from the controller device 20, the implant device 20 applies the stimulus to the predetermined stimulation part in accordance with the received instruction.

According to an example of the present aspect, the status f the physiological signals to be detected can be divided into a plurality of patterns, and the stimuli to be applied can be made different among the patterns, respectively.

### Explanation on Numerals

1 stimulus application system, 10 implant device , 11 transmission/reception unit, 12 power supply unit, 13 stimulus circuit unit, 14 sensor unit, 15 processor unit, 20 controller device, 21 transmission/reception unit, 22 control unit, 23 storage unit, 24 communication unit, 25 power source unit, 221 reception unit, 222, 222' stimulus determination unit, 223 instruction transmission unit, 225 trial stimulation unit, 226 evaluation unit.

## Claims

1. A stimulus application system comprising:
an implant device implanted in an object body which is an animal including a human being, and
a controller device arranged outside of the object body that can wirelessly communicate with the implant device, wherein
the implant device comprises:
a circuitry for detection which repeatedly detects an electrical signal representing a physiological signal at a predetermined part in the object body at a predetermined time interval,
a circuitry for transmission/reception which transmits detection information representing the detected electrical signal, and receives, from the controller device, a stimulation instruction representing a stimulus to be applied to the object body, and
a circuitry for application which applies an electrical stimulus to a predetermined part in the object body, on the basis of the stimulation instruction received by the circuitry for transmission/reception, and
the controller device comprises:
a circuitry for reception which receives the detection information transmitted by the implant device,
a circuitry for determination which acquires information regarding time variation of the physiological signal at the predetermined part in the object body by using the received detection information, and determines type of stimulus to be applied to the object body by the implant device on the basis of the information regarding the time variation, and
a circuitry for transmission which transmits a stimulation instruction representing the determined stimulus to the implant device.

2. An implant device implanted in an object body which is an animal including a human being, and being communicated wirelessly to a controller device arranged outside of the object body, wherein the implant device comprises:
a circuitry for detection which repeatedly detects an electrical signal representing a physiological signal at a predetermined part in the object body at a predetermined time interval,
a circuitry for transmission/reception which transmits detection information representing the detected electrical signal, and receives, from the controller device, a stimulation instruction representing a stimulus to be applied to the object body, and
a circuitry for application which applies an electrical stimulus to a predetermined part in the object body, on the basis of the stimulation instruction received by the circuitry for transmission/reception.

3. An implant device according to claim 2, wherein
the circuitry for detection repeatedly detects an electrical signal representing a physiological signal at a predetermined part in the object body at a predetermined time interval, for 2ⁿ times (n being a natural number of 1 or more), and stores the detection result, and
the circuitry for transmission/reception transmits the detection result of the electrical signal detected for the 2ⁿ times, as detection information representing time variation of the detected electrical signal.

4. An implant device according to claim 2, wherein
the circuitry for detection repeatedly detects an electrical signal representing a physiological signal at a predetermined part in the object body at a predetermined time interval, and
the circuitry for transmission/reception transmits the detection information representing the electrical signal continuously.

5. An implant device according to claim 2, further comprising:
an circuitry for determination in the implant device which transforms time-domain information representing time variation of the detected electrical signal to frequency-domain information, and determines type of a stimulus to be applied to the object body on the basis of the frequency-domain information, wherein
the circuitry for application applies an electrical stimulus to a predetermined part in the object body, on the basis of the stimulus details determined by the implant device circuitry for determination.

6. An implant device according to claim 5, wherein
the circuitry for determination in the implant device transforms each information regarding time variation of a physiological signal at the predetermined part in the object body, obtained for a plurality of times in the past, to frequency-domain information by the fast Fourier transformation method, and synthesizes the result of frequency-domain information obtained plurality of times in the past, and applies the electrical stimulus to the predetermined part in the object body with reference to the synthesized frequency-domain information and the stimulus setting information.

7. A controller device arranged outside of an object body which is an animal including a human being, and at a position to communicate wirelessly with an implant device implanted in the object body, wherein
the implant device repeatedly detects an electrical signal representing a physiological signal at a predetermined part in the object body at a predetermined time interval, and wirelessly transmits detection signal representing the detected electric signal, and
the controller device comprises:
a circuitry for reception which receives the detection information transmitted by the implant device,
a circuitry for determination which acquires information regarding time variation of the physiological signal at the predetermined part in the object body by using the received detection information, and determines type of a stimulus to be applied to the object body by the implant device on the basis of the information regarding the time variation, and
a circuitry for transmission which transmits a stimulation instruction representing the determined stimulus to the implant device.

8. A controller device according to claim 7, wherein
the information regarding the time variation is time-domain information representing the time variation of the electrical signal detected by the implant device, and
the circuitry for determination transforms the acquired information regarding the time variation to frequency-domain information, and determines type of a stimulus to be applied to the object body by the implant device on the basis of the frequency-domain information.

9. A controller device according to claim 8, wherein the circuitry for determination transforms the acquired information regarding the time variation to frequency-domain information by the fast Fourier transformation.

10. A controller device according to claim 7, further comprising:
a circuitry for trial stimulation which transmits stimulation instructions representing a plurality of prospective stimuli having different type of stimuli to the implant device, at each predetermined time, and
a circuitry for evaluation which evaluates the effect of the transmitted stimulation instruction as trial stimulation, using the detection information received by the implant device after performing this trial stimulation, wherein
an evaluation result of each of the stimulation instructions corresponding to the plurality of prospective stimuli is subjected to a predetermined process relating to the determination of the stimulation instruction.

11. A controller device according to claim 10, wherein
each of the plurality of prospective stimuli is a stimulus by a periodic electrical signal, and at least one of the following is mutually different among the prospective stimuli:
- amplitude of the stimulus,
- duration of the stimulus,
- pulse width of the stimulus, and
- frequency of the stimulus.

12. A controller device according to claim 7, wherein
the implant device repeatedly detects an electrical signal representing a physiological signal at a predetermined part in the object body at a predetermined time interval, and wirelessly transmits the detection information representing the detected electrical signal continuously that the electrical signal is detected, and
the circuitry for determination of the controller device accumulates and stores the detection information received from the implant device until the electrical signal detection result of the 2ⁿ times of detection is acquired, the stored electrical signal detection result of the 2ⁿ times of detection being information regarding time variation of the physiological signal at the predetermined part in the object body; transforms the information regarding time variation of the physiological signal to frequency-domain information by the fast Fourier transformation method; and determines type of stimulus to be applied to the object body by the implant device on the basis of the frequency-domain information.

13. A controller device according to claim 12, wherein
the circuitry for determination transforms each information regarding time variation of a physiological signal at the predetermined part in the object body, obtained for a plurality of times in the past, to frequency-domain information by the fast Fourier transformation method, synthesizes the frequency-domain information of the plurality of times in the past, and determines type of a stimulus to be applied to the object body by the implant device on the basis of the synthesized frequency-domain information.

14. A controller device according to claim 7, which holds stimulus setting information including a plurality of mutually different stimulus applying conditions respectively associated with information representing the details of corresponding stimuli,
the stimulus applying condition including a condition regarding the synthesized frequency-domain information,
wherein the circuitry for determination acquires information regarding time variation of the physiological signal at the predetermined part in the object body by using the received detection information, and determines type of a stimulus to be applied to the object body by the implant device with reference to the frequency-domain information obtained by transforming the acquired information, and the stimulus setting information.

15. A controller device according to claim 14, wherein
the circuitry for determination transforms each information regarding time variation of a physiological signal at the predetermined part in the object body, obtained for a plurality of times in the past, to frequency-domain information by the fast Fourier transformation method, synthesizes the frequency-domain information of the plurality of times in the past, and determines type of a stimulus to be applied to the object body by the implant device with reference to the synthesized frequency-domain information, and the stimulus setting information

16. A method for controlling a controller device arranged outside of an object body which is an animal including a human being, and at a position to communicate wirelessly with an implant device implanted in the object body, the implant device repeatedly detecting an electrical signal representing physiological signal at a predetermined part in the object body at a predetermined time interval, wherein
a circuitry for reception receives detection information representing a detection result of the electrical signal, transmitted by the implant device,
a circuitry for determination acquires information regarding time variation of the physiological signal at the predetermined part in the object body by using the received detection information, and determines type of a stimulus to be applied to the object body by the implant device on the basis of the information regarding the time variation, and
a circuitry for transmission transmits a stimulation instruction representing the determined stimulus to the implant device.

17. A computer readable and non-transitory recording medium which stores a program for a controller device arranged outside of an object body which is an animal including a human being, that can wirelessly communicable with an implant device embedded in the object body, the implant device repeatedly detecting an electrical signal as a physiological signal at a predetermined part in the object body at a predetermined time interval
the program functioning the controller device **as:**
a circuitry for reception which receives detection information representing a detection result of the electrical signal, transmitted by the implant device
a circuitry for determination which receives information regarding time variation of the physiological signal at the predetermined part in the object body, and determines type of a stimulus to be applied to the object body by the implant device on the basis of the information regarding the time variation, and
a circuitry for transmit a stimulation instruction representing the determined stimulus to the implant device.
